# EUROPEAN PATENT APPLICATION

(11) **EP 4 063 866 A1**
(43) Date of publication of application: **28.09.2022**
(21) Application number: 21165362.1
(22) Date of filing: 26.03.2021
(51) Int. Cl.: G01N 33/68, C12P 21/00

(54) **METHOD FOR GLYCOSYLATION PROFILING TO DESCRIBE FUNCTIONAL CHARACTERISTICS OF A BIOLOGIC MOLECULE**

(71) Applicant: Ares Trading S.A., 1170 Aubonne (CH)
(72) Inventor: MASTRANGELI, Renato, 00012 Guidonia Montecelio (IT); SATWEKAR, Abhijeet, 00012 Guidonia Montecelio (IT)
(74) Representative: Merck Serono S.A. Intellectual Property

(57) **Abstract**

The glycosylation profile of a biologic molecule describes important aspects of such biologic molecule. The characterization of the glycosylation profile of a biologic molecule is an important part of the critical quality attributes in the manufacturing and analytical processes for biologic molecules. As such described herein are methods to determine and characterize the glycosylation profile of a biologic molecule. In particular, the method uses one or more indices to describe the glycosylation profile as a finger-print of such biologic molecule.

## Description

### Field of the Invention

The invention described herein relates to methods to determine and characterize the glycosylation profile of a biologic molecule. In particular, the method uses one or more indices to describe the glycosylation profile of such biologic molecule. The characterization of the glycosylation profile of a biologic molecule being an important part of the critical quality attributes in the manufacturing and analytical processes for biologic molecules.

### Background of the Invention

Recombinant glycoproteins (monoclonal antibodies, cytokines, hormones, Fc-fusion molecules) are an important class of biotherapeutics. Today numerous antibodies and other glycoproteins are under active development by pharmaceutical companies.

With respect to these biotherapeutics, glycosylation heavily affects their molecular properties in terms of stability, solubility, clearance rate, efficacy, immunogenicity and safety. The glycosylation profile of a given drug is heavily dependent on the host cell line and is very sensitive to cell culture conditions. Furthermore, the final glycosylation profile is dependent on the purification processes.

Glycosylation is therefore one of the critical quality attributes (CQAs) in a glycosylated biopharmaceutical drug that must be tightly monitored for example during development of such glycosylated biopharmaceutical drug as well as in comparability studies following manufacturing process changes for its production, and in the biosimilar development (comparability with the reference medicinal product). At each glycosylation site, a distribution of glycoforms is generated, resulting in glycoproteins with high heterogeneity at each site and an increased complexity and heterogeneity at overall glycoprotein level when multiple glycosylation sites are present.

To ensure safety and efficacy, regulatory agencies require a stringent glycan analysis as an essential part of quality control strategy.

The assessment of the glycan profile is based on the complete characterization of a reference in house standard that is then used as a comparator with the production batches. Currently, for release tests, this comparison is generally performed by using the glycan release method. However, glycan release suffers of from several limitations especially when considering glycoproteins with multiple glycosylation sites in which specific site(s) is/are relevant for efficacy or when considering antibody-fused to glycoproteins. By using this methodology, relevant site-specific information including that related to O-glycans is lost, and the quantitation may be impacted by an incomplete release or recovery of the glycans. Furthermore, the resulting released N-glycan mixture may contain undesired N-glycans e.g. released by host cell proteins or glycan released by non-canonical glycosylation sites, if any is present at a detectable level.

As such, a major drawback of the release method, when applied to glycoproteins containing multiple glycosylation sites, is the averaged amount of N-glycan species which is not suitable to assess relevant changes which have a functional impact. Furthermore, sites which are partially glycosylated are not taken in consideration resulting in an out-of-scope designation in case a change in occupancy is present. Moreover, the overall glycan-release method loses value when analyzing immune-cytokines or antibodies fused to a soluble receptor as no information of Fc glycans which play a pivotal role for the Fc effector functions can be retrieved.

In addition, there is currently a lack of standardized metrics for observing shifts in glycoform distributions occurring during cell culture experiments. Indexes have been developed to describe glycosylation patterns. Examples of such indexes are the Z-number, and the A-index which are calculated based on the overall released glycans. Z number is reported and calculated in the pharmacopoeia for Follitropin concentrated solution. Z-number represents the hypothetical charge number (Z) to characterize protein glycosylation. The Z number is defined as the sum of the products of the relative areas (%) of the N-glycan species, each multiplied by the corresponding charge (number of sialic acid). The A-index represents the hypothetical antennarity index (A). The antennarity index is defined as the sum of the products of the relative areas (%) of the N-glycan species, each multiplied by the corresponding antennae number (n). Such indexes were recently applied also at each FSH site by using the glycopeptide methodology, and when averaged, these values were found to be similar to that obtained by glycan release method.

However, such indexes, have their limitations and should not be used to describe the percent glycosylation of an entire population. Frequently, the indexes are related only to one glycosylation site present in the Fc region of an antibody for example. Therefore, there is a need, due to the limitations of such indexes, for an improved method to describe the glycosylation profile of a biologic molecule.

### Summary of the Invention

The present invention provides methods to overcome the above described limitations and provide a more suitable method to determine and describe the glycosylation profile of a biologic molecule. Such methods as described herein better visualize and characterize the glycoprotein content in terms of sialic acid and antennarity at each site, and in each subunit/domain and or in the whole molecule. The method utilizes data acquired by for example LC-MS based analytical methods and subsequently uses a new indices system for describing the glycosylation of a biologic molecule.

In one embodiment of the present invention there is provided a method to determine a glycosylation identity of a biologic molecule comprising:
a. submitting the biologic molecule to LC-MS analysis,
b. from the results obtained in step a), calculate the value of at least three of indices selected from Sialylation Index, Sialylation Extent, NANA 2,6%, NANA 2,3%, NGNA%, O-acetyl-NANA%, Antennarity Index, site occupancy extent (SOE), Core Fucosylation extent, Antennary fucose Index, Galactosylation Index, G0 for molecules containing the Fc region, G1 for molecules containing the Fc region, G2 for molecules containing the Fc region , alpha Gal Index, LAC repeat Index, Mannose Index, Hybrid index, Bisecting Index, and Neutral Index, N-acetylglucosamine index (GlcNac), and Core-xi extent for O-glycans; and
c. arrange the calculated values obtained in step b),
wherein the arranged values describe the glycosylation identity of the biologic molecule.

Such combination and method to describe the glycosylation of a biologic molecule provides an improved method which can be used in various further methods such as for determination of differences between glycosylation as a result in process changes, for example to be used in process validation. Also it is possible to better identify critical quality attributes of biologic molecule and potentially determine identity of the biologic molecule. Such further methods include further embodiments of the invention described herein.

In such other embodiment of the present invention there is provided a method of determining biosimilarity of a biologic molecule with a reference biologic molecule comprising: a.) submitting the biologic molecule to LC-MS analysis, b.) from the results obtained in step a), calculate the value of at least three of indices selected from Sialylation Index, Sialylation Extent, NANA 2,6%, NANA 2,3%, NGNA %, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and c.) determine the deviation of calculated values obtained in step c) with the values for the reference biologic molecule, wherein the biologic molecule is considered similar with respect to its glycosylation if the deviations determined in step c) are within a preset range and is considered not similar with respect to its glycosylation if the deviations determined in step c) are outside of the preset range. Biosimilarity is often understood as a biologic molecule having the same amino acid structure and the same functional characteristics. A difference in glycosylation profile while being of the same biologic molecule (i.e. having the same amino acid structure) could indicate that the biologic molecule that is analyzed in a sample is a biosimilar of the reference biologic molecule.

In yet another embodiment of the present invention there is provided a method of establishing comparability within a process for preparing a biologic molecule comprising: a.) preparing a first biologic molecule in a first batch according to the process for preparing the biologic molecule, b.) preparing a second biologic molecule in a second batch according to the process for preparing the biologic molecule, wherein the process in step a) and step b) comprise the identical process steps, c.) purify separately the first and second biologic molecules from mixtures containing the first and second biologic molecules obtained in step a) and step b) to obtain a purified first biologic molecule and a purified second biologic molecule, d.) submit each of the purified first and second biologic molecules to LC-MS analysis, e.) from the results obtained in step d), calculate the value for at least three of indices selected from Sialylation Index, Sialylation Extent, NANA 2,6%, NANA 2,3%, NGNA %, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and f.) determine the deviation in calculated values for each of the selected indices in step e) between those for the first biologic molecule and the second biologic molecule, wherein the first and second biologic molecule are considered the same if the deviations determined in step f) are within a preset range.

In yet another embodiment of the present invention there is provided a method for determining comparability within a process of a biologic molecule with a reference biologic molecule, comprising: a.) preparing a biologic molecule in a batch according to the process for preparing the biologic molecule, b.) purify the biologic molecule from a mixture containing the biologic molecule obtained in step a) to obtain a purified biologic molecule, c.) submit the purified biologic molecule to LC-MS analysis, d.) from the results obtained in step c), calculate the value for at least three of indices selected from Sialylation Index, Sialylation Extent, NANA 2,6%, NANA 2,3%, NGNA%, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and e.) determine the deviation of the calculated values for each of the selected indices in step d) with reference values for the indices for the biologic molecule, wherein the biologic molecule is considered the same as a reference biologic molecule if the deviations determined in step e) are within a preset range.

In yet another embodiment of the present invention there is provided a method of establishing comparability between different processes for producing the same biologic molecule comprising: a.) preparing a first biologic molecule according to a first process for preparing the biologic molecule, b.) preparing a second biologic molecule according to a second process for preparing the biologic molecule, wherein the process in step a) and step b) comprise at least one different process step, c.) purify separately the first and second biologic molecules from mixtures containing the first and second biologic molecules obtained in step a) and step b) to obtain a purified first biologic molecule and a purified second biologic molecule, d.) submit each of the purified first and second biologic molecules to LC-MS analysis, e.) from the results obtained in step d), calculate the value for at least three of indices selected from Sialylation Index, Sialylation Extent, NANA 2,6%, NANA 2,3%, NGNA %, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and f.) determine the deviation in calculated values for each of the selected indices in step e) between those for the first biologic molecule and the second biologic molecule, wherein the first and second biologic molecule are considered the same if the deviations determined in step f) are within a preset range and the first and second process produce biosimilar biologic molecules.

Another embodiment of the present invention provides a method determining functional characteristics of a biologic molecule comprising: a.) determining the glycosylation identity of a biologic molecule according to the method of the first embodiment above (i.e. the steps of a.) submitting the biologic molecule to LC-MS analysis, b.) from the results obtained in step a), calculate the value of at least three of indices selected from Sialylation Index, Sialylation Extent, NANA 2,6%, NANA 2,3%, NGNA %, O-acetyl-NANA%, Antennarity Index, site occupancy extent (SOE), Core Fucosylation extent, Antennarity fucose Index, Galactosylation Index, G0 for molecules containing the Fc region, G1 for molecules containing the Fc region, G2 for molecules containing the Fc region, alpha Gal Index, LAC repeat Index, Mannose Index, Hybrid index, Bisecting Index, and Neutral Index, N-acetylglucosamine index (GlcNac), and Core-xi extent for O-glycans; and c.) arrange the calculated values obtained in step b), wherein the arranged values describe the glycosylation identity of the biologic molecule), b.) comparing the values obtained for the selected indices with reference values for such indices which reference values either alone or in combination correlate to a functional characteristic of a biologic molecule, and c.) determine one or more functional characteristics of the biologic molecule selected from immunogenicity risk, drug-receptor interaction, mAb effector function(s), allosteric modulation of binding sites, bioavailability, pharmacokinetics, biologic molecule stability, and solubility.

### Detailed description

In order to provide an improved method to describe the glycosylation profile of a biologic molecule the present invention describes various methods which describe the glycosylation of such biologic molecule. Such improved methods using a number of indexes allow for analysis, comparison and release of biologic molecules manufactured for use in humans as an active pharmaceutical ingredient. The methods described herein allow for the identification of inter process variations or their absence, allow batch comparisons and determination of a biologic molecule with a particular glycosylation profile for batch release and/or determination of biosimilarity.

The methods as used herein make use of various indices to describe glycosylations that can occur on biologic molecules. Such indices include for example the Sialylation Index, the Sialylation Extent, NANA 2,6%, NANA 2,3%, NGNA%, O-acetyl-NANA %, Antennarity Index, site occupancy extent (SOE), Core Fucosylation extent, Antennary fucose Index, Galactosylation Index, alpha Gal Index, LAC repeat Index, Mannose Index, Hybrid index, Bisecting Index, and Neutral Index, N-acetylglucosamine index (GlcNac) and Core-xi extent (only for O-glycans). In addition, certain further indices are used wherein the biologic molecule contains an Fc region, such indices are for example the G0, G1, and G2 indices. Each of these indices can be calculated for a specific site within the molecule to represent the glycosylation at such specific site. Alternatively, in certain embodiments of the present invention's methods, some indices can also be calculated as a total for the entire molecule, such as for example Sialylation Index, Galactosylation Index, alpha Gal Index, Mannose Index and N-acetylglucosamine index (GlcNac Index). In addition, a total NANA (S_{NANA}) index or total NGNA (S_{NGNA}) index can be calculated as well in any of the methods of the present invention. For such indices either a site-specific index can be calculated, a total molecule index can be calculated or both. Such total molecule indexes can also be of use in determining or describing functional characteristics of the biologic molecule such as for example its immunogenicity and clearance.

A minimum of three indices are used in any one of the methods as described in the present invention. The indices are calculated and determined based on experimental data collected using any suitable analytical method such as for example LC-MS (a combination of any type of liquid chromatography to separate components and Mass spectroscopy to determine the identity of such components). More specifically the Sialylation Index (SI) is the ponder average number of sialic acid present and is obtained considering each glycoform estimated in terms of its relative percentage (%) and number (x) of sialic acid residues present. Thus the Sialylation index can be expressed as S-index = (Σ (% AnGnSx ^{∗} x) + Σ (% FAnGnSx ^{∗} x)) / ((Σ(% AnGnSx) + Σ (% FAnGnSx) )= (Σ (% AnGnSx ^{∗} x)+ Σ (% FAnGnSx ^{∗} x)) / 100.

The Sialylation Extent (SE) is the ponderal average sialylation degree obtained considering each glycoform estimated in terms of its relative percentage (%), the number (x) of sialic acid residues present e.g.(0- 4), and the total number of sialic acid that a given glycoform could potentially accommodate (xmax=n) (e.g. biantennary xmax = 2, triantennary xmax = 3, tetra-antennary xmax = 4). SE represents therefore a measure of the extent of sialic acid "end-capping" on terminal galactoses of the sugar chains, as a proportion of the total amount of end-capping present. As such, the SE index can be represented by S-Extent = Σ (% AnGnSx ^{∗} x/n) + Σ (% FAnGnSx ^{∗} x/n). Where: AnGnSx is a glycoform with antennarity =n, galactose number =n, and sialic acid number = x; n can range from 2 to 4; for bi-antennary n=2, tri-antennary n=3, tetra-antennary n=4; x can range from 0 to n, and is dependent on the sialic acid content on the glycoform; for a-sialylated x =0, mono-sialylated x=1, di-sialylated x=2, tri-sialylated x=3 and tetra-sialylated x=4). The term FAnGnSx considers the fucosylated glycoforms.

NANA 2,6% and NANA 2,3 % are the relative % of Neu5Ac / N-acetylneuraminic acid with α2,6 linkage or with α2,3 linkage respectively within the total sialylation. NGNA % represents the relative % of Neu5Gc / N-Glycolylneuraminic acid within the total sialylation. O-acetyl-NANA % represents the relative % of O-acetylated Neu5Ac / N-acetylneuraminic acid within total sialylation

Antennarity Index (AI) is the ponderal average number of antennae present and is obtained considering each glycoform estimated in terms of its relative percentage (%) and the number (n) of antennae present. Such as A-index = (Σ (% AnGnSx ^{∗}n) + Σ (% FAnGnSx ^{∗} n )) / (Σ (% AnGnSx) + Σ (% FAnGnSx)) = (Σ (% AnGnSx ^{∗}n) + Σ (% FAnGnSx ^{∗} n)) / 100.

Futher, the Core Fucosylation extent (cFE) represents the relative % of core fucosylation. While the Antennae fucose Index (aFI) represents the ponderal average number of fucose residues present in the antennae. Also, the Galactosylation index (GI) represents the ponderal average number of peripheral exposed galactose residues within the total glycan distribution.

Where the biologic molecule contains an Fc region, the G0 represents the complex type glycans with 0 galactose residues present. The G1 represents the complex type glycans with 1 galactose residue, and the G2 represents the complex type glycans with 2 galactose residues.

Moreover, the α Gal index (αGI) represents the ponderal average number Galactose in an alpha -1-3 linkage. The LAC repeat (LI) is the ponderal average number of LAC repeats (Gal-GlcNac) within the total glycan distribution. The Mannose index (MI) is the ponderal average number of High mannose structures (M5-M9) within the total glycan distribution. While the Hybrid index (HI) is the ponderal average number of hybrid structures within the total glycan distribution. The Bisecting index (BI) is the ponderal average number of bisecting structures within the total glycan distribution, and the Neutral Index (NI) is the ponderal average number of neutral glycan structures within the total glycan distribution.

Another important index is the Site occupancy extent (SOE) which has been defined herein as the relative percentage (%) of glycan present in a specific site. SOE =100 the site is fully occupied by the glycan. SOE = 0 not occupied.

Further, galactosylation index and the N-acetylglucosamine index (GlcNac) may provide information regarding functional aspects of the biologic molecule such as for example its clearance from the body of the individual having taken a medication containing the biologic molecule. The presence of terminal Gal and GlcNac residues suggest a higher affinity for the asialoglycoprotein receptor in liver, which suggest an impact for the Gal and GlcNac indices towards the clearance of the biologic molecule. Moreover, also the α2,6 sialylation index (NANA/NGNA) but not the α2,3 sialylation index can contribute to clearance due to higher exposure of the underlining galactose residue to environment that allow to bind asialoglycoprotein receptor. The mannosylation index suggest a contribute to clearance and or immunogenicity due to interaction with with mannose receptor. NGNA and α-gal indices are representative of the content of xenogenic glycan residues in the biologic molecule, and suggest a contribute on clearance due to pre-existing anti-xenogenic antibodies in humans; as well as to potential clinical risks in terms of safety and immunogenicity.

The glycosylation profile of a biologic molecule provides important information regarding its critical quality attributes and is potentially linked to its activity. A known glycosylation profile allows to determine comparability and compatibility of the biologic molecule with like biologic molecules. As such a description of its glycosylation profile of such biologic molecule allows for determining inter and intra-process comparability. Furthermore, it is useful in batch release processes and maintaining quality for a biologic molecule produced at large for use in treatments of humans. The methods described herein require a minimum of three indices to describe the biologic molecule and its glycosylation profile.

As such a method of the present invention to determine a glycosylation identify of a biologic molecule comprises: a.) submitting the biologic molecule to LC-MS analysis, and subsequently in step b.) calculate from the results obtained in step a), the value of at least three of indices selected from Sialylation Index, Sialylation Extent, NANA 2,6%, NANA 2,3%, NGNA%, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and c.) arrange the calculated values obtained in step b) such that the arranged values describe the glycosylation identity of the biologic molecule.

The LC-MS analysis as used herein can be any known method of using a chromatographic separation technique followed by any known technique to determine the identity of the separated analytes obtained from the separation technique. As such Mass Spectrometry (MS) is an exemplary method for such determination of analyte identity that can be used with a liquid chromatography technique. In large scale production in the pharmaceutical industry LC-MS is an accepted analytical method to determine glycosylation of proteins or parts of proteins. Frequently this method comprises an affinity type liquid chromatography technique which can also be HPLC. The collected fractions are subjected to Mass Spectroscopy to determine their identity.

The method as described herein could also include a step of first purifying a biologic molecule of interest. As such the method includes potential additional steps wherein first the biologic molecule of interests is purified from a "crude" sample obtained from a process producing the biologic molecule. For example, when the methods of the present invention are used in process quality checks it is possible that the biologic molecule of interest at that point in the production process is not yet purified. A sample at such step could first be purified to obtain a sample for which the glycosylation profile can be determined. Any known process to purify the particular biologic molecule (often dependent on the biologic molecule itself) can be used in such purification step.

Also is provided a method of determining biosimilarity of a biologic molecule with a reference biologic molecule comprising the same steps as in the method described herein before including a.) submitting the biologic molecule to LC-MS analysis, and b.) from the results obtained in step a), calculate the value of at least three of indices selected from Sialylation Index, Sialylation Extent, NANA 2,6%, NANA 2,3%, NGNA%, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans). It further comprises the step c.) of determining the deviation of calculated values obtained in step c) with the values for the reference biologic molecule. The biologic molecule can be considered biosimilar to the reference molecule if the deviations determined in step c) are within a preset range and is considered not similar if the deviations determined in step c) are outside of the preset range.

In a similar manner the method of the present invention can be used to determine differences or the absence thereof between the same biologic molecule produced by either the same method (but at different times) or produced using a different or modified production method. Such analytical methods aid in providing information relating to the critical quality attributes, and necessary information for batch release. In addition, such analytical method allow comparing the obtained biologic molecule from two processes, whether modified or carried out at a different time or location or both and as such verify that the biologic molecule has the same glycosylation profile despite of these differences in producing processes of the same biologic molecule.

One such method can be to establish comparability within a process for preparing a biologic molecule comprising: a.) preparing a first biologic molecule in a first batch according to the process for preparing the biologic molecule, b.) preparing a second biologic molecule in a second batch according to the process for preparing the biologic molecule, wherein the process in step a) and step b) comprise the identical process steps, c.) purify separately the first and second biologic molecules from mixtures containing the first and second biologic molecules obtained in step a) and step b) to obtain a purified first biologic molecule and a purified second biologic molecule, d.) submit each of the purified first and second biologic molecules to LC-MS analysis, e.) from the results obtained in step d), calculate the value for at least three of indices selected from Sialylation Index, Sialylation Extent, NANA 2,6%, NANA 2,3%, NGNA%, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and f.) determine the deviation in calculated values for each of the selected indices in step e) between those for the first biologic molecule and the second biologic molecule. The first and second biologic molecule are considered the same if the deviations determined in step f) are within a preset range.

Likewise, a method for determining comparability within a process of a biologic molecule with a reference biologic molecule is provided. Such method comprises the steps of a.) preparing a biologic molecule in a batch according to the process for preparing the biologic molecule, b.) purify the biologic molecule from a mixture containing the biologic molecule obtained in step a) to obtain a purified biologic molecule, c.) submit the purified biologic molecule to LC-MS analysis, d.) from the results obtained in step c), calculate the value for at least three of indices selected from Sialylation Index, Sialylation Extent, NANA 2,6%, NANA 2,3%, NGNA %, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and e.) determine the deviation of the calculated values for each of the selected indices in step d) with reference values for the indices for the biologic molecule. In such method the biologic molecule is considered the same as a reference biologic molecule if the deviations determined in step e) are within a preset range. Such result of being the same for the biologic molecule can be used as a method to verification of the production process or as a measure for batch release of the obtained product biologic molecule.

Thus, in any of these methods batch release for a production batch can be provided wherein the biologic molecule for which the glycosylation profile is determine is from a sample of a production batch and when such biologic molecule and the reference biologic molecule are considered biosimilar. In a similar manner (bio)similarity with respect to its glycosylation can be determined. Included is wherein the biologic molecule is considered not similar with respect to its glycosylation if the deviations determined in these methods are outside of the preset range. Biosimilarity is often understood as a biologic molecule having the same amino acid structure and the same functional characteristics. A difference in glycosylation profile while being of the same biologic molecule (i.e. having the same amino acid structure) could indicate that the biologic molecule that is analyzed in a sample is a biosimilar of the reference biologic molecule.

In this context the present invention can also be used whether two different processes produce the same biologic molecule. Such information is helpful when making changes to an existing process in order to verify that a modified process remains producing the same biologic molecule. Frequently, health authorities would require such information in order to approve such manufacturing change for the approved pharmaceutical biologic molecule. It could likewise be used to determine successful tech transfer of a manufacturing process from one facility to another production facility. Successful tech transfer is established when the biologic molecule produced at both facilities has the same glycosylation profile. This glycosylation profile being determined according to a method of the present invention.

Accordingly, there is provided a method of establishing comparability between different processes for producing the same biologic molecule comprising: a.) preparing a first biologic molecule according to a first process for preparing the biologic molecule, b.) preparing a second biologic molecule according to a second process for preparing the biologic molecule, wherein the process in step a) and step b) comprise at least one different process step, c.) purify separately the first and second biologic molecules from mixtures containing the first and second biologic molecules obtained in step a) and step b) to obtain a purified first biologic molecule and a purified second biologic molecule, d.) submit each of the purified first and second biologic molecules to LC-MS analysis, e.) from the results obtained in step d), calculate the value for at least three of indices selected from Sialylation Index, Sialylation Extent, NANA 2,6%, NANA 2,3%, NGNA%, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and f.) determine the deviation in calculated values for each of the selected indices in step e) between those for the first biologic molecule and the second biologic molecule. The first and second biologic molecule are considered the same if the deviations determined in step f) are within a preset range and the first and second process produce biosimilar or the same biologic molecules.

The identity of the biologic molecule through its glycosylation profile may also impact functional aspects of the biologic molecule. As such the present invention also allows for a method determining functional characteristics of a biologic molecule comprising: a.) determining the glycosylation identity of a biologic molecule as described herein followed by the steps of, b.) comparing the values obtained for the selected indices with reference values for such indices which reference values either alone or in combination correlate to a functional characteristic of a biologic molecule, and c.) determine one or more functional characteristics of the biologic molecule selected from immunogenicity risk, drug-receptor interaction, mAb effector function, allosteric modulation of binding sites, effector function, bioavailability, biologic molecule stability, and solubility.

In such method can further comprise comparing the glycan profile of a sample biologic molecule with a reference glycan profile for the biologic molecule and determine whether the biologic molecule is from the same source and/or process as the reference biologic molecule.

### Examples:

### Example 1: Glycopeptide mapping

Reduction, alkylation and enzymatic digestion. Individual batches of innovator and biosimilar glycoproteins were concentrated to approximately 1 mg/mL using Amicon Ultracel 3k centrifugal filters. Following concentration, 200 µL of protein was then suspended in 200 µL of denaturation buffer containing 8M guanidine-HCL, 130 mM Tris-HCI and 1 mM EDTA at pH 7.6, and reduction was performed by adding 20 µL of 500 mM DTT and stirring for 30 minutes at 37°C. The samples were subsequently alkylated by adding 25 µL of 500 mM IAM and stirring in the dark for 30 minutes at room temperature. The buffer containing the reduced and alkylated samples was then exchanged for a digestion buffer containing 2 M urea and 50 mM Tris-HCI at pH 8.0 by using Amicon Ultracel 3k centrifugal filters. The proteins in the samples were then digested at 37°C for 4 hours using chymotrypsin, with an enzyme:substrate ratio of 1:20.

### Example 2: Hydrophilic interaction chromatography and mass spectrometry analysis

Chymotrypsin digested protein was analysed using a Synapt G1 mass spectrometer (Waters, Milford, MA,USA) equipped with an Acquity UPLC system (Waters, Milford, MA, USA). The peptides were separated on an Acquity glycan BEH amide UPLC column (1.7µm, 2.1 x 150 mm), owing to the high resolving separation for glycosylated peptides and the enhanced peptide retention and separation of peptides from glycopeptides attained with this method [38], and eluted with a mixture of 0.1% TFA in water and 0.1% TFA in ACN. A 30-55% gradient of 0.1% TFA in water over 60 minutes with 0.2 mL/min flow rate was used to separate the glycopeptides. Separated site-specific glycopetide populations (different peptides, various attached glycans) as well as the different site-specific glycopeptides populations (same peptide, various attached glycans) were identified and quantified by mass spectrometry.

Mass spectrometry was performed using the MS^{E} function for dataset acquisition in the data independent mode. MS^{E} function uses an intelligent approach and acquires alternating scans with low and high collision energies to obtain precursor ion information, as well as collision-induced dissociation (CID) fragmentation data. The instrument was operated with the following parameters: capillary voltage 3 kV, sampling cone 28 V, extraction cone 4 V, source temperature 100°C, desolvation temperature 350°C, cone gas flow 50 L/H, desolvation gas flow 800 L/H, and scan range 100-2000 m/z.

The mass spectrometry data were analyzed by MassLynx 4.1 software (Waters, Milliford, MA, USA). The identity of the N-glycopeptides was manually assigned (S1 Table), and the m/z specific ion counts were determined by the extraction ion chromatogram (XIC). Identified glycopeptides were grouped as per the N-glycan site, and the relative distribution of the site-specific N-glycan species was calculated based on ion counts. This strategy allows comparison of the relative distribution of the N-glycan species attached on the site-specific glycopeptide. Moreover, the use of the unique glycopeptide for the corresponding N-glycan site does not significantly create an ionization bias. As the ionization is mostly due to the contribution of the peptide portion, this is the same for each site-specific glycopeptide population (same peptide, different glycans). LC-MS methods with chymotrypsin digest have been reported that detect and quantitate glycan variants. The resulting data from the glycopeptide analysis was further used to calculate the indexes for describing the glycosylation profile of the biologic molecule.

### Example 3: determination of indices for an antibody/Fc fusion protein.

Following a procedure as described in examples 1 and 2, to obtained data, that allowed for the calculation of the indices for an antibody/Fc fusion protein is shown in the following tables as an example of comparability. These indexes would facilitate a better elaboration on comparability during the manufacturing process changes and also provide a tighter criterion towards the assessments. The differences are clearly captured and highlighted in grey shading.

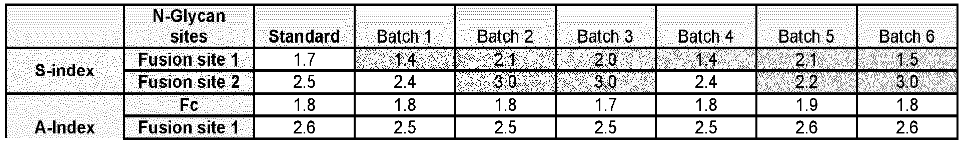

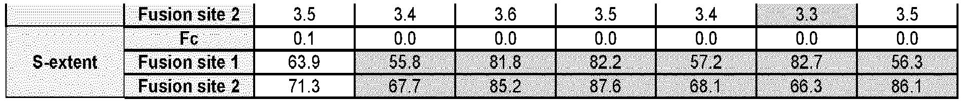

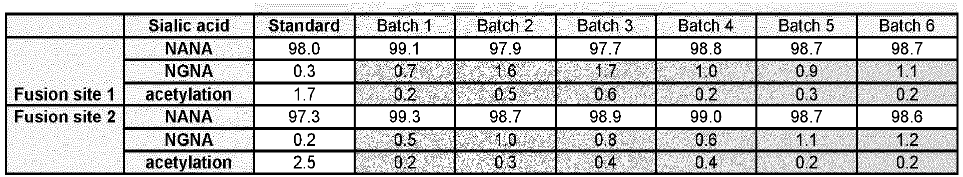

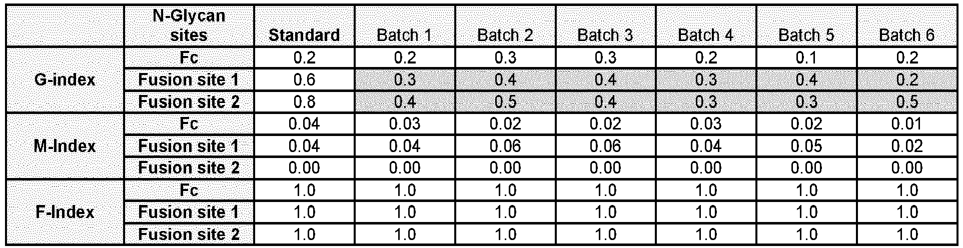

**Example 4:** Comprehensive N-glycosylation pattern indexing for comparing differences between batches of a glycoprotein.

The analysis as described in examples 1 and 2 has been used and the obtained data was subsequently used to calculate the indices for elaborating the N-glycosylation profiles. These indices were for sialylation, fucosylation, mannosylation, galactosylation and antennarity. All of these are critical for elucidating the bioactivity and efficacy of the glycosylated therapeutic product. These indices facilitate easy interpretation and provides high accuracy in defining biosimilarity in terms of glycosylation of the product.

The index values for the biosimilarity study are provided in the following table. The differences are clearly captured and highlighted in grey shading.

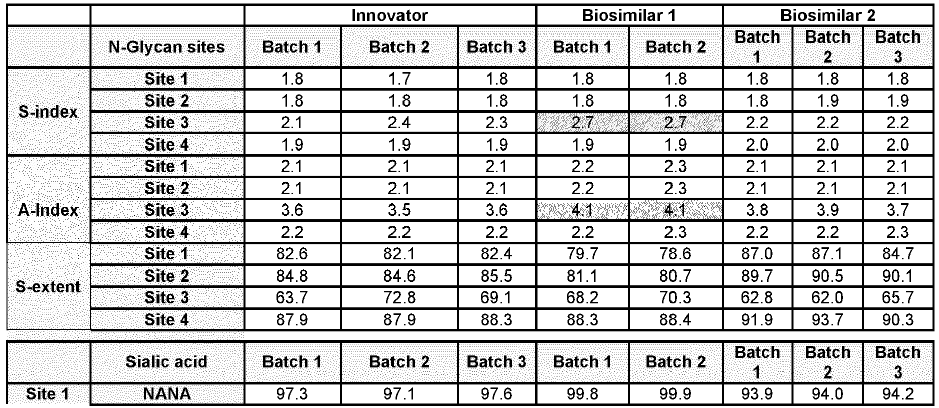

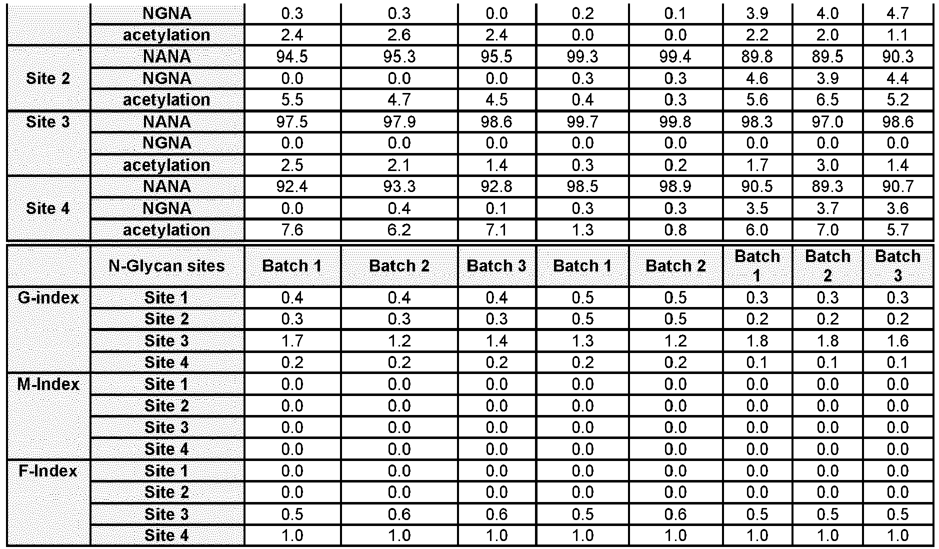

## Claims

1. A method to determine a glycosylation identity of a biologic molecule comprising:
a. submitting the biologic molecule to LC-MS analysis,
b. from the results obtained in step a), calculate the value of at least three of indices selected from Sialylation Index, Sialylation Extent, NANA 2,6%, NANA 2,3%, NGNA%, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and
c. arrange the calculated values obtained in step b),
wherein the arranged values describe the glycosylation identity of the biologic molecule.

2. The method according to claim 1, wherein the biologic molecule is first purified from a mixture containing the biologic molecule.

3. The method according to claim 1, wherein the arranged values are a standardized metric for describing the glycosylation identity of the biologic molecule.

4. The method according to claim 1, wherein the arranged values describe a finger-print profile of the biologic molecule.

5. A method of determining biosimilarity of a biologic molecule with a reference biologic molecule comprising:
a. submitting the biologic molecule to LC-MS analysis,
b. from the results obtained in step a), calculate the value of at least three of indices selected from Sialylation Index, Sialylation Extent, NANA 2,6%, NANA 2,3%, NGNA%, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and
c. determine the deviation of calculated values obtained in step c) with the values for the reference biologic molecule,
wherein the biologic molecule is considered biosimilar if the deviations determined in step c) are within a preset range and is considered not similar if the deviations determined in step c) are outside of the preset range.

6. A method of establishing comparability within a process for preparing a biologic molecule comprising:
a. preparing a first biologic molecule in a first batch according to the process for preparing the biologic molecule,
b. preparing a second biologic molecule in a second batch according to the process for preparing the biologic molecule, wherein the process in step a) and step b) comprise the identical process steps,
c. purify separately the first and second biologic molecules from mixtures containing the first and second biologic molecules obtained in step a) and step b) to obtain a purified first biologic molecule and a purified second biologic molecule,
d. submit each of the purified first and second biologic molecules to LC-MS analysis,
e. from the results obtained in step d), calculate the value for at least three of indices selected from Sialylation Index, Sialylation Extent, NANA 2,6%, NANA 2,3%, NGNA%, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and
f. determine the deviation in calculated values for each of the selected indices in step e) between those for the first biologic molecule and the second biologic molecule,
wherein the first and second biologic molecule are considered the same if the deviations determined in step f) are within a preset range.

7. A method for determining comparability within a process of a biologic molecule with a reference biologic molecule, comprising:
a. preparing a biologic molecule in a batch according to the process for preparing the biologic molecule,
b. purify the biologic molecule from a mixture containing the biologic molecule obtained in step a) to obtain a purified biologic molecule,
c. submit the purified biologic molecule to LC-MS analysis,
d. from the results obtained in step c), calculate the value for at least three of indices selected from Sialylation Index, Sialylation Extent, NANA 2,6%, NANA 2,3%, NGNA%, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and
e. determine the deviation of the calculated values for each of the selected indices in step d) with reference values for the indices for the biologic molecule,
wherein the biologic molecule is considered the same as a reference biologic molecule if the deviations determined in step e) are within a preset range.

8. The method according to claim 7, further comprising batch release of the batch of which the biologic molecule is a sample when the biologic molecule and the reference biologic molecule are considered biosimilar.

9. The method according to claim 7, wherein the method is determining biosimilarity of the biologic molecule with the reference biologic molecule.

10. The method of claim 9, wherein the biologic molecule is considered not biosimilar in terms of glycosylation if the deviations determined in step e) are outside of the preset range.

11. A method of establishing comparability between different processes for producing the same biologic molecule comprising:
a. preparing a first biologic molecule according to a first process for preparing the biologic molecule,
b. preparing a second biologic molecule according to a second process for preparing the biologic molecule, wherein the process in step a) and step b) comprise at least one different process step,
c. purify separately the first and second biologic molecules from mixtures containing the first and second biologic molecules obtained in step a) and step b) to obtain a purified first biologic molecule and a purified second biologic molecule,
d. submit each of the purified first and second biologic molecules to LC-MS analysis,
e. from the results obtained in step d), calculate the value for at least three of indices selected from Sialylation Index, Sialylation Extent, NANA 2,6%, NANA 2,3%, NGNA%, O-acetyl-NANA %, Antennarity Index, site occupancy extent, Core Fucosylation extent, Antennarity fucose Index, Galactosylation Index, G0 only for molecule containing the Fc region, G1 only for molecule containing the Fc region, G2 only for molecule containing the Fc region, alpha Gal Index, LAC repeat Index, Mannose Index, Hybrid index, Bisecting Index, and Neutral Index, N-acetylglucosamine index (GlcNac), and Core-xi extent (only for O-glycans), and
f. determine the deviation in calculated values for each of the selected indices in step e) between those for the first biologic molecule and the second biologic molecule,
wherein the first and second biologic molecule are considered the same if the deviations determined in step f) are within a preset range and the first and second process produce biosimilar biologic molecules.

12. A method determining functional characteristics of a biologic molecule comprising:
a. determining the glycosylation identity of a biologic molecule according to the method of claim 1,
b. comparing the values obtained for the selected indices with reference values for such indices which reference values either alone or in combination correlate to a functional characteristic of a biologic molecule, and
c. determine one or more functional characteristics of the biologic molecule selected from immunogenicity risk, drug-receptor interaction, mAb effector functions, allosteric modulation of binding sites, bioavailability, pharmacokinetics, biologic molecule stability, and solubility.

13. The method according to claim 12, wherein when the functional characteristic of the biologic molecule is glycan profile the method for further comprises comparing the glycan profile of a sample biologic molecule with a reference glycan profile for the biologic molecule and determine whether the biologic molecule is from the same source and/or process as the reference biologic molecule.

14. The method according to claim 13, wherein if there is a difference between the glycan profile of the sample biologic molecule and the reference glycan profile, the sample biologic molecule is considered a counterfeit biologic molecule.
